# EUROPÄISCHE PATENTSCHRIFT

(11) **EP 1 206 281 B1**
(45) Veröffentlichungstag und Bekanntmachung des Hinweises auf die Patenterteilung: **30.06.2004**
(21) Anmeldenummer: 00947994.0
(22) Anmeldetag: 25.07.2000
(51) Int. Cl.: A61K 31/4709, A61K 47/02

(54) **FORMULIERUNG ENTHALTEND MOXIFLOXACIN UND NATRIUMCHLORID**
FORMULATION CONTAINING MOXIFLOXACIN AND SODIUM CHLORIDE
FORMULATION COMPRENANT MOXIFLOXACIN ET CHLORURE DE SODIUM

(30) Priorität: 06.08.1999 DE 19937116
(43) Veröffentlichungstag der Anmeldung: 22.05.2002
(73) Patentinhaber: Bayer HealthCare AG, 51368 Leverkusen (DE)
(72) Erfinder: KÜHN, Bernd, D-51061 Köln (DE); MAHLER, Hans-Friedrich, D-51061 Köln (DE); EISELE, Michael, D-51469 Bergisch Gladbach (DE)
(86) Internationale Anmeldenummer: PCT/EP2000/007098
(87) Internationale Veröffentlichungsnummer: WO 2001/010465

(56) Entgegenhaltungen:
- WO-A-00/18386

## Beschreibung

Die vorliegende Erfindung betrifft eine wäßrige Formulierung, die Moxifloxacin-Hydrochlorid und Natriumchlorid enthält und das Verfahren ihrer Herstellung.

Moxifloxacin (INN - International Nonproprietary Name) ist ein Antibiotikum aus der Klasse der Chinoloncarbonsäuren der folgenden Formel:

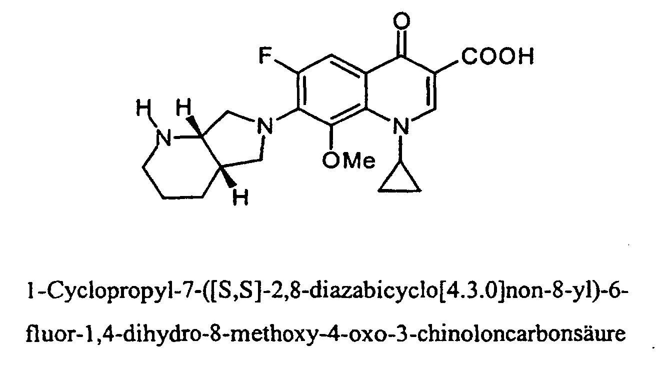

Es ist ein hochwirksames antiinfektives Mittel und wurde erstmals beschrieben in der EP-A-0 350 733. Die EP-A-0 350 733 beschreibt jedoch keine pharmazeutische Zubereitungen, die zur parenteralen Verabreichung geeignet sind. Insbesondere zur Behandlung von Patienten auf Intensivstationen, die zur oralen Aufnahme nicht befähigt sind, bedarf es jedoch einer solchen parenteral verabreichbaren Infusionslösung.

Für die Formulierung verträglicher Infusionslösungen wird die Angleichung der Osmolalität an die physiologischen Bedingungen des Organismus gefordert (Sucker/Fuchs/Speiser; Pharmazeutische Technologie). Bei stärkerer hypo- oder hyperosmotischer Abweichung kann es zu Erythrozytenschädigung bzw. Gewebereizungen kommen. Bei i.v.-Gabe stärker hypoosmotischer Lösungen tritt Hämolyse, bei Zufuhr größerer Mengen hyperosmotischer Lösungen tritt Plasmolyse auf. Hypoosmotische Lösungen enthalten weniger gelöste Moleküle oder Ionen als im Blut oder der Gewebsflüssigkeit vorhanden sind. In diesem Fall muß durch Zugabe von Isotonisierungsmitteln eine Isotonisierung erfolgen (Bauer/Frömming/Führer; Pharmazeutische Technologie). Als isotonisch wird dabei ein Bereich von 270 bis 350 mOsmol/kg als zweckmäßig erachtet.

Handelsübliche isotonische Lösungen sind z.B. eine 5%ige Glucoselösung oder eine 0,9%ige Kochsalzlösung.

Die EP-A-0534860 beschreibt Formulierungen des Chinoloncarbonsäure-Antibiotikums Sparfloxacin mit Monocarboxyl-Polyhydroxysäuren oder deren Lactone, wie z.B. Ascorbinsäure und mit Glucose oder Glycerol als isotonisierendem Zusatz. Die Erfindung beruht auf der Löslichkeitsverbesserung von Sparfloxacin durch Monocarboxyl-Polyhydroxysäuren zur Erzielung von verträglichen, isotonen oder hypertonen Formulierungen geeigneter Konzentration.

Die US-A-5,563,149 beschreibt die Formulierung wässriger Lösungen von Pyridoncarbonsäuren und deren Ester und Salze als Antibiotika als anwendungsfertige Injektions- oder Infusionslösungen oder Injektions- oder Infusionskonzentrate. Angaben über Isotonisierungszusätze oder zur Tonizität der Formulierungen werden nicht gemacht. Ziel dieser Erfindung ist die Löslichkeitsverbesserung der beschriebenen Pyridoncarbonsäuren.

In EP-A-0507851 werden Formulierungen beschrieben, die Chinoloncarbonsäure-Metallion-Säurekomplexe beinhalten. Durch Zusatz von mehrvalenten Metallionen in Form von Magnesium-, Calcium-, Mangan-, Zink, Kadmium,-Aluminium-, Ceroder Eisenionen wird in Folge von Komplexbildung bei neutralem pH-Wert eine erhöhte Löslichkeit des Wirkstoffs gefunden. Es wird beschrieben, dass solche Formulierungen chemisch und physikalisch stabil auch in Gegenwart von Glucose zur Isotonisierung sind und eine verbesserte Verträglichkeit durch Erzielen eines neutralen pH-Wertes besitzen.

US 5,811,130 beschreibt Metallion-Komplexe mit Danofloxacin, bei denen insbesondere Magnesium- und Zinkionen zur Komplexbildung verwendet werden und die eine deutlich erhöhte Löslichkeit des Danofloxacin bewirken. Es werden Formulierungen hoher Wirkstoffkonzentration zur subcutanen Injektion beschrieben, die erst durch die verbesserte Wasserlöslichkeit der Metallion-Wirkstoff-Komplexe erreicht werden können.

Weiterhin wird in US 5,084,276 die Verwendung von Chinoloncarbonsäure-Metallionen-Komplexen z.B. mit Magnesium-, Calcium-, Mangan-, Zink-, Cadmium-, Eisen-(II)- und Eisen-(III)- oder Cer-(IV)-Ionen zur Komplexierung der Wirkstoffe Temafloxacin, Toxyfloxacin oder Pefloxacin gezeigt, wobei die Wirkstoffkomplexe zusammen mit Hilfstoffen zur Reduktion der Venenirritation verwendet werden. Die beschriebenen Formulierungen zur parenteralen Infusion sind mit Glukose isotonisiert.

Im Rahmen der Entwicklungsarbeiten zu Moxifloxacin wurde überraschenderweise gefunden, dass der Weg der Isotonisierung durch Zusatz von 5% handelsüblicher Glucose oder anderer Zucker oder Zuckeralkohole, wie 2,5% Glycerol, bei Moxifloxacin zu instabilen Lösungen führt. Diese Instabilität äußert sich im Auftreten von subvisuellen Partikeln in der Lösung, deren Anzahl oberhalb des von den Pharmakopöen (USP XXIII, BP93) zulässigen Bereiches liegen. Hierbei bilden sich im Verlauf der Lagerung braun gefärbte, amorphe Partikel, die oft erst nach 4-8 Wochen Lagerung bei 40°C auftreten und im Verlauf der Lagerung zahlenmäßig weiter zunehmen. Bei Raumtemperatur oder Kühlschranklagerung ist die Bildung dieser Partikel verlangsamt. Die Erfinder fanden, dass die Partikelbildung durch eine dreifach-Wechselwirkung zwischen Moxifloxacin bzw. seinen Salzen, Eisen und Zucker bzw. Zuckeralkoholen, wie Glycerol, hervorgerufen wird. Dieser Sachverhalt war überraschend, da ähnliche Phänomene bei der Formulierung von parenteralen Chinoloncarbonsäureformulierungen bislang nicht bekannt waren und insbesondere die EP 0507851, US 5,811,130 und US 5,084,276 die Wechselwirkung von mehrwertigen Metallionen mit Chinoloncarbonsäuren zur Stabilisierung und Löslichkeitserhöhung ausnutzen. Das Antibiotikum Ciprofloxacin toleriert beispielsweise wesentlich höhere Eisenkonzentrationen.

Da Eisen als Element ubiquitär vorkommt und insbesondere in dem Einsatzstoff Glucose, in dem es komplex gebunden sein kann, vorliegt, ist eine solche Formulierung nur mit großem analytischen und qualitätssichernden Aufwand herstellbar. Zusätzlich ist die Verwendung von Stahl für die Herstellanlagen problematisch und nur ausgewählte Stähle und überwachte Materialien dürfen mit der Lösung in Kontakt geraten. Weiterhin erfordert eine solche Formulierung grundsätzlich das Vorliegen extrem eisenarmer Wirkstoffqualitäten,. die nur mit großem Aufwand herstellbar sind. Lösungen, die einen Eisengehalt oberhalb 20 ppb enthalten, zeigen bei Moxifloxacin einen zeitlich stark anwachsenden Gehalt an Partikeln, so dass die erforderliche pharmazeutische Qualität der Formulierungen nach der Herstellung für den erforderlichen Haltbarkeitszeitraum nicht aufrechterhalten werden kann. Darüberhinaus sind glucoseisotonisierte Formulierungen in verschiedenen Bereichen der klinischen Anwendungen als sehr nachteilig eingestuft, da sie eine zusätzliche Belastung für den Energiehaushalt des Patienten darstellen können und insbesondere bei der Behandlung von Diabetikern besonders berücksichtigt werden müssen.

Bei parenteralen wäßrigen Formulierungen von Hydrochloriden von Chinoloncarbonsäuren besteht aufgrund deren schlechten Löslichkeitseigenschaften in Gegenwart von NaCI im allgemeinen das Problem das Mittel mit einem vertretbaren Infusionsvolumen zu verabreichen. Neben der oben beschriebenen Möglichkeit der Löslichkeitserhöhung durch Metallkomplexbildung, sind auch verschiedene Möglichkeiten der Salzbildung beschritten worden.

So beschreibt die EP-A-0219784 Infusionslösungen von Ciprofloxacin mit physiologisch verträglichen Säuren. Es wird auch eine Formulierung von 75mg/500 ml Ciprofloxacin (0,015 % M/V), 0,203 ml/500 ml 1M Salzsäure (entsprechend einem molaren Verhältnis Ciprofloxacin/Salzsäure von 1,0 zu 0,9) und 4,5 g/500 ml (0,9 %) Natriumchlorid als Isotonisierungszusatz beschrieben. Diese Wirkstoffkonzentration entspricht in etwa der Sättigungslöslichkeit des Wirkstoffs in der angegebenen Formulierung bei Raumtemperatur. Höhere Konzentrationen von Ciprofloxacin in Gegenwart von Salzsäure und isotonischer Mengen NaCI lassen sich aufgrund der schlechten Löslichkeit des Ciprofloxacins bzw. seines Hydrochlorids nicht realisieren. Bei einer üblichen Dosierung des Ciprofloxacins von 2 bis 3-mal täglich 100 bis 400 mg pro Dosierung ergeben sich somit inakzeptable Infusionsvolumina von ca. 1,3 l bis 8 l täglich. Aufgrund der bei einer gegebenen Dosierung sehr hohen Menge an Infusionsflüssigkeit verwendet man daher bevorzugt wäßrige Formulierungen des besser löslichen Ciprofloxacin-Laktats.

Es wurde durch die Erfinder zunächst überraschend gefunden, dass Arzneiformulierungen von Moxifloxacin-Hydrochlorid, die mit Natriumchlorid isotonisiert sind, keine Empfindlichkeit gegen Eisenionen aufweisen. Die Löslichkeitseigenschaften des Wirkstoffs Moxifloxacin in Form seines Hydrochlorides sind in Gegenwart von Natriumchlorid allerdings extrem schlecht, so dass anfängliche Entwicklungsversuche solcher Formulierungen nach Auftreten von Ausfällungen . zunächst aufgegeben wurden. Überraschenderweise wurde jedoch gefunden, dass akzeptable Formulierungen von Moxifloxacin-Hydrochlorid unter Isotonisierung. mit Kochsalz hergestellt werden können, wenn bestimmte enge Konzentrationsbereiche für Wirkstoff und isotonisierendes Agens eingehalten werden.

Gegenstand der Erfindung sind somit wäßrige Formulierungen die 0,04 % bis 0,4 % M/V Moxifloxacin-Hydrochlorid, berechnet als Moxifloxacin, und von 0,4 % bis 0,9 % M/V Natriumchlorid enthalten.

Eine wäßrige Formulierung bedeutet, dass die Bestandteile der Formulierung in Wasser vorliegen.

Die Angabe "% M/V" bedeutet Masse in g pro 100 ml Volumen, also g/100 ml.

Die wäßrige Formulierung der Erfindung enthält bevorzugt 0,08 % bis 0,32 % M/V Moxifloxacin-HCI, berechnet als Moxifloxacin und besonders bevorzugt 0,1 % M/V bis 0,2 % M/V Moxifloxacin-HCI, (berechnet als Moxifloxacin). Ganz besonders bevorzugt ist eine Formulierung mit etwa 0,16 % M/V Moxifloxacin-HCI, berechnet als Moxifloxacin, entsprechend 400 mg/250 ml.

Die wäßrige Formulierung, der Erfindung enthält 0,4 % bis 0,9 % M/V Natriurnchlorid, be vorzugt 0,5 % bis 0,9 % M/V Natriumchlorid, bevorzugter 0,7 % bis 0,9 % M/V Natrium. chlorid, und ganz besonders bevorzugt ist ein Zusatz an Natriumchlorid von etwa 0,8 % M/V.

Für einen Dosierungsbereich von 100 mg bis 1000 mg kann bei Wirkstoffkonzentrationen von 0,04 % bis 0,4 % M/V Moxifloxacin-Hydrochlorid also eine Angleichung des osmotischen Drucks an die physiologischen Verhältnisse durch Zusatz von 0,4 % bis 0,9 % Kochsalz erreicht werden. Hierzu ist es notwendig, die Sättigungslöslichkeit des Wirkstoffes in Gegenwart der verschiedenen Konzentrationen an Kochsalz bei einer Temperatur von 5 °C zu berücksichtigen und Wirkstoffkonzentration sowie Natriumchlorid-Konzentration in ein optimales erfindungsgemäßes Verhältnis zu bringen. Die Sättigungslöslichkeit des Wirkstoffs in Anwesenheit der zur Isotonisierung erforderlichen Mengen an Natriumchlorid gemessen bei 5°C darf dabei nicht überschritten werden. Damit ist gewährleistet, dass auch durch kurzfristige Kühllagerung keine Ausfällungen des Wirkstoffs durch Überschreiten der Sättigungslöslichkeiten vorkommen.

Für Lösungen oberhalb einer Wirkstoffkonzentration von ca. 0,2 % M/V Moxifloxacin-HCl können mit Natriumchlorid keine ideal isotonisierten Lösungen hergestellt werden, da die Löslichkeit der Substanz zu gering wird. Da es für intravenös verabreichte Lösungen jedoch rasch durch den Blutstrom zu einer Verdünnung der Infusionslösung kommt, die schmerzfrei geschieht, so ist eine bestmögliche Angleichung der Tonizität an die physiologischen Verhältnisse ausreichend. Der optimalen Verträglichkeit solcher Lösungen kann durch entsprechende Anpassung der Infusionsgeschwindigkeit Rechnung getragen werden (Lit. Sucker/Fuchs/Speiser, "Pharmazeutische Technologie", Thierne Verlag 1991, S. 460ff).

Als isotone Lösung wird eine Lösung bezeichnet, die einen osmotischen Druck von ca. 270 bis 330 mOsmol aufweist. Dies entspricht einer Kochsalzlösung der Konzentration von ca. 0,8 % bis 0,9 % M/V Demgegenüber trägt Moxifloxacin-HCl zur Isotonisierung kaum bei. Es zeigt sich überraschend, dass sich in Gegenwart dieser NaCl-Konzentration Moxifloxacin-HCl ausreichend und stabil löst, so dass eine derartige Formulierung als Formulierung für die parenterale Verabreichung geeignet ist.

**Tabelle 1:**

| Löslichkeit von Moxifloxacin-HCl in Gegenwart von Natriumchlorid bei 5°C | | | | | | | | | | |
|---|---|---|---|---|---|---|---|---|---|---|
| NaCl % M/V | 0 | 0,1 | 0,2 | 0,3 | 0,4 | 0,5 | 0,6 | 0,7 | 0,8 | 0,9 |
| Löslichkeit Moxifloxacin-HCl % M/V | 1,8 | 1,1 | 0,7 | 0,5 | 0,4 | 0,3 | 0,2 | 0,2 | 0,2 | 0,1 |

Besonders bevorzugt ist eine Dosierung von 200 mg bis 800 mg Moxifloxacin, entsprechend Konzentrationen von 0,08 % bis 0,32 % M/V Moxifloxacin. Die Einstellung des osmotischen Drucks gelingt mit 0,9 % bis 0,4 % M/V Natriumchlorid. Ganz besonders bevorzugt ist eine Dosierung von 400 mg Moxifloxacin in Form einer etwa 0,16 %igen M/V Lösung von Moxifloxacin. Diese wird mit ca. 0,8 % M/V Natriumchlorid isotonisiert.

Es wurde ferner gefunden, dass die Herstellung solcher Lösungen von Moxifloxacin mit Natriumchlorid erhebliche Zeit in Anspruch nimmt, wenn auf herkömmliche Weise der Wirkstoff Moxifloxacin-Hydrochlorid in einer Vorlage des in Wasser gelösten Natriumchlorids eingetragen und zur Lösung gebracht werden soll. Hierbei wird eine Rührzeit von mehreren Stunden gefordert, um eine klare Lösung der Formulierung zu erreichen und anschließende Wirkstoffverluste durch Abtrennung des Wirkstoffs bei Filtrationsschritten zu vermeiden. Bei Anwendung eines Verfahrens, bei dem zuerst der Wirkstoff in Wasser gelöst wird und erst anschließend das Natriumchlorid zugegeben wird, gelingt die Lösungsherstellung innerhalb weniger Minuten und ermöglicht eine rationelle Fertigung im industriellen Maßstab.

Gegenstand der Erfindung ist daher auch ein Verfahren zur Herstellung der wäßrigen Formulierung der Erfindung, worin zunächst eine Lösung des Moxifloxacin-Hydrochlorids in Wasser hergestellt wird und anschließend Natriumchlorid zugegeben und gelöst wird.

Zweckmäßig erfolgt die Herstellung der wäßrigen Formulierung von Moxifloxacin-Hydrochlorid dadurch, dass eine Lösung von Moxifloxacin-Hydrochlorid in Wasser mit einer Konzentration des Moxifloxacin-Hydrochlorids (bezogen auf die Menge des Moxifloxacins) von mehr als 0,4% M/V bis 2,4% M/V, also ein Konzentrat des Moxifloxacin-Hydrochlorids in Wasser, mit einer wäßrigen Lösung, die Natriumchlorid enthält, auf die erfindungsgemäße Konzentration des Moxifloxacin-Hydrochlorids verdünnt wird.

Gegenstand der Erfindung ist somit ferner die Verwendung einer wäßrigen Lösung von Moxifloxacin-Hydrochlorid in Wasser mit einer Konzentration des Moxifloxacin-Hydrochlorids (bezogen auf die Menge des Moxifloxacins) von mehr als 0,4 % M/V bis 2,4 % M/V (im folgenden gelegentlich als Wirkstoffkonzentrat bezeichnet) zur Herstellung eines Arzneimittels zur parenteralen Verabreichung, sowie ein Kombinationspräparat, das eine wäßrige Lösung von Moxifloxacin-Hydrochlorid in Wasser mit einer Konzentration des Moxifloxacin-Hydrochlorids (bezogen auf die Menge des Moxifloxacins) von mehr als 0,4% M/V bis 2,4% M/V und eine wäßrige Lösung, die Natriumchlorid enthält, getrennt voneinander umfaßt, wobei die konzentrierte wäßrige Lösung des Moxifloxacin-Hydrochlorids mit der mitgeliefeiten wäßrigen Lösung, die Natriumchlorid enthält, zweckmäßig vom behandelnden Arzt bzw. vom Pflegepersonal selbst gemischt wird.

Die konzentrierte wäßrige Lösung des Moxifloxacin-Hydrochlorid enthält von 0,4% M/V bis 2,4% M/V Moxifloxacin-Hydrochlorid (bezogen auf die Menge des Moxifloxacin). Die maximale Konzentration der wäßrigen Lösung ist durch die Sättigungslöslichkeit von ca. 2,4 % M/V begrenzt. Vorzugsweise enthält das Wirkstoffkonzentrat 1,0 bis 2,0 % M/V Moxifloxacin-Hydrochlorid (bezogen auf die Menge des Moxifloxacin), besonders bevorzugt 2,0% M/V Moxifloxacin-Hydrochlorid (bezogen auf die Menge des Moxifloxacin). Das Wirkstoffkonzentrat wird in geeignete Behältnisse abgefüllt und geeignet sterilisiert. Die Behältnisse können sowohl aus Glas wie auch aus Kunststoff bestehen. Dabei können die Behältermaterialien Substanzen enthalten, die dem Inhalt einen besonderen Schutz verleihen, wie z.B. einen Lichtschutz oder einen Sauerstoffschutz.

Durch Mischen mit Natriumchlorid enthaltenden Lösungen wird die Verdünnung des Wirkstoffkonzentrates auf Anwendungskonzentrationen des Moxifloxacins entsprechend der erfindungsgemäßen wäßrigen Formulierung hergestellt. Gegebenenfalls können die Lösungen zur Verdünnung des Wirkstoffkonzentrates neben Natriumchlorid auch andere Salze mit Natrium, Kalium, Calcium, Magnesium etc., wie Chloride, Carbonate, Sulfate, Acetate, Gluconate, Lactate, Malate sowie weitere auf dem Gebiet der parenteralen Applikationsformen übliche Hilfsstoffe etc. enthalten, sofern die Entstehung einer homogenen Infusionslösung sichergestellt ist. Es können zur Verdünnung des Wirkstoffkonzentrates auch gängige, im Handel erhältliche Infusionsträgerlösungen eingesetzt werden.

Die wäßrige Formulierung der Erfindung dient zweckmäßig als Arzneimittel zur parenteralen Applikation insbesondere als Arzneimittel zur Prävention oder Behandlung bakterieller Infektionen. Die parenterale Applikation schließt z.B. die intravenöse, intraarterielle, subkutane, intramuskuläre sowie die intraperitoneale Verabreichung ein, wobei der intravenösen Verabreichung die größte Bedeutung zukommt. Als Dosis werden zweckmäßig 400 mg Wirkstoff bezogen auf die Betainform für eine 1x tägliche intravenöse Infusion erachtet. Das täglich verabreichte Infusionsvolumen sollte 200 bis 250 ml nicht übersteigen. Daraus ergibt sich bei einer Wirkstoffmenge von 400 mg eine Wirkstoffkonzentration von ca. 0,16 % M/V entsprechend 400mg/250 ml.

Die wäßrige Arzneimittelformulierung der Erfindung kann zusätzlich zu den erfindungsgemäß verwendeten Inhaltsstoffen weitere auf dem Gebiet der parenteralen Applikationsformen übliche Hilfsstoffe, wie z.B. Säuren und Basen zur Einstellung des pH-Wertes sowie übliche Konservierungsmittel und Antioxidantien enthalten.

Es ist überaus überraschend, dass aus den erfindungsgemäßen Formulierungen des Moxifloxacins selbst bei mehrwöchiger Lagerung bei niedrigen Temperaturen, die zur Ausfällung des Wirkstoffes führten, durch einfaches Erwärmen auf Raumtemperatur wieder klare partikelfreie Lösungen entstehen: Eine Reifung der Ausfällung im Sinne der Ausbildung von stabilen, groben Kristallstrukturen wurde nicht beobachtet, so dass die Formulierung nach der vorliegenden Erfindung als stabil und sicher für eine Vermarktung eingestuft werden kann.

Weiterhin ist es für die Herstellanlagen ausreichend, Stahlqualitäten allgemeiner pharmazeutischer Qualität einzusetzen. Die erfindungsgemäßen Formulierungen sind nämlich überraschender Weise im Gegensatz zu Formulierungen des Moxifloxacins, die Zucker oder Zuckeralkohole aufweisen, gegenüber der Gegenwart von Eisen stabil. Die unten beschriebenen Versuche mit gezieltem Zusatz von Eisenionen zur Lösung zeigen, dass auch bei Mengen von 1 ppm Eisen, also der 50fachen Menge gegenüber dem für eine Glucoseformulierung zulässigen Grenzwert, keine Partikelbildung in der Lösung auftritt. Die erfindungsgemäßen Formulierungen sind daher als Formulierungen für die parenterale Verabreichung aufgrund ihrer Stabilität und einfachen Herstellbarkeit. hervorragend geeignet.

Die Erfindung wird im Hinblick auf die folgenden Beispiele näher erläutert.

### Beispiele

### Vergleichsbeispiel 1 :

Isotone Moxifloxacin-Formulierung, 0,2 % M/V (400 mg/200 ml), isotonisiert mit Glucose 5%

| | |
|---|---|
| Moxifloxacin-HCl | 0,2 % M/V * |
| Glucose Monohydrat | 5,0 % M/V |
| Wasser f. Injektionszwecke | 94,8 % M/V |

| | |
|---|---|
| * Menge bezogen auf das Moxifloxacin in der Betainform | |

In einem Ansatzbehälter aus rostfreiern Stahl pharmazeutischer Qualität wird das Wasser vorgelegt und Moxifloxacin-Hydrochlorid unter Rühren darin gelöst. In die Lösung des Wirkstoffs wird die Glucose (handelsübliche Qualität, ca 380 ppm Fe) zugegeben und gelöst. Nach Filtration über ein 0,2 µm Sterilfilter wird zu 200 ml in Infusionsflaschen abgefüllt, verschlossen und bei 121°C für 20 min im Autoklaven sterilisiert. Die fertige Lösung enthält ca. 25 ppb Eisen.

Das hergestellte Produkt zeigt nach Lagerung bei 40 °C folgende Werte zum Partikelgehalt:

| Lagerbedingungen | Partikel > 25 µm/ml (Grenzwert max. 2/ml; USP XXIII) | | |
|---|---|---|---|
| | Partie A | Partie B | Partie C |
| Anfangsprüfung | 0,6/ml | 0,6/ml | 0,7/ml |
| 4 Wochen 40 °C | 5,6/ml | 4,2/ml | 4,3/ml |

Das Produkt ist nicht stabil und zeigt bereits nach 4 Wochen Lagerung bei 40°C einen unzulässigen Anstieg der Partikelwerte der die Anforderungen der Arzneibücher nicht erfüllt.

### Vergleichsbeispiel 2:

Moxifloxacin-Formulierung 0,4% M/V (400 mg/100 ml), Tonizitätsangleich mit Natriumchlorid 0,3%

| | |
|---|---|
| Moxifloxar-in-HCl | 0,4 % M/V* |
| Natriumchlorid . | 0,3 % M/V |
| Wasser f. Injektionszwecke | 99,3 % M/V |

| | |
|---|---|
| * Menge bezogen auf das Moxifloxacin in der Betainform | |

In einem Ansatzbehälter aus Edelstahl wird das Wasser vorgelegt und Moxifloxacin-Hydrochlorid unter Rühren darin gelöst. Der Lösung des Wirkstoffs wird Natriumchlorid zugegeben und gelöst. Nach Filtration über ein 0,2 µm Sterilfilter wird zu 100 ml in Infusionsflaschen abgefüllt, verschlossen und bei 121 °C für 20 min im Autoklaven erhitzt.

Die Lösung weist eine Osmolalität von etwa 100 mOsmol/kg auf, und ist daher hypoton, was bei üblicher Verabreichungsgeschwindigkeit parenteraler Lösungen zu Hämolyse und schmerzhafter Verabreichung führt.

### Beispiel 1: Moxifloxacin-Formulierung 0,16 % M/V (400 mg/250 ml)

| | |
|---|---|
| Moxifloxacin-HCl | 0,16 % M/V * |
| Natriumchlorid | 0,8 % M/V |
| Wasser f. Injektionszwecke | 99,44 % M/V |
| Osmolalität: 281 mOsmol/kg | |

| | |
|---|---|
| * Menge bezogen auf das Moxifloxacin in der Betainform | |

In einem Ansatzbehälter aus Glas wird das Wasser vorgelegt und Moxifloxacin-Hydrochlorid unter Rühren darin gelöst. In die Lösung des Wirkstoffs wird Natriumchlorid zugegeben und gelöst. Dem Ansatz wird eine Fe-III-chlorid-Lösung zugegeben. Nach Filtration über ein 0,2 µm Sterilfilter wird zu 250 ml in Infusionsflaschen abgefüllt, verschlossen und bei 121°C für 20 min. im Autoklaven erhitzt.

Das hergestellte Produkt zeigt nach Lagerung bei Raumtemperatur und bei 40 °C folgende Werte zum Partikelstatus:

### Eisengehalt der Lösung 540 ppb

| Lagerbedingungen | Partikel ≥ 25 µm/ml (Grenzwert max. 2/ml; USP XXIII) |
|---|---|
| Anfangsprüfung | 0,00 |
| 4 Wochen 40°C | 0,13 |
| 8 Wochen 40°C | 0,17 |
| 8 Wochen 25°C | 0,00 |

Die Lösung erweist sich als lagerstabil und nicht empfindlich gegen Eisenionen.

### Eisengehalt der Lösung < 10 ppb

| Lagerbedingungen | Partikel ≥ 25 µm/ml (Grenzwert max. 2/ml; USP XXIII) |
|---|---|
| Anfangsprüfung | 0,07 |
| 12 Wochen 40 °C | 0,19 |
| 78 Wochen 25°C | 0,07 |
| 78 Wochen 30°C | 0,15 |

Die Lösung erweist sich als lagerstabil und nicht empfindlich gegen Eisenionen.

### Beispiel 2: Moxifloxacin-Formulierung 0,1% M/V (100 mg/100.ml)

| | |
|---|---|
| Moxifloxacin-HCl | 0,1% M/V* |
| Natriumchlorid | 0,9% M/V |
| Wasser f. Injektionszwecke | 99,0% M/V |
| Osmolalität: 313 mOsmol/kg | |

| | |
|---|---|
| * Menge bezogen auf das Moxifloxacin in der Betainform | |

In einem Ansatzbehälter aus rostfreiem Stahl pharmazeutischer Qualität wird das Wasser vorgelegt und Moxifloxacin-Hydrochlorid unter Rühren darin gelöst. Der Lösung des Wirkstoffs wird Natriumchlorid zugegeben und gelöst. Nach Filtration über ein 0,2 µm Sterilfilter wird zu 100 ml in Infusionsflaschen abgefüllt, verschlossen und bei 121 °C für 20 min im Autoklaven erhitzt.

Das hergestellte Produkt zeigt nach Lagerung bei Raumtemperatur und bei 40 °C folgende Werte zum Partikelgehalt:

| Lagerbedingungen | Partikel ≥ 25 µm/ml (Grenzwert max. 2/ml; USP XXIII) |
|---|---|
| Anfangsprüfung | 0,03 |
| 4 Wochen 40°C | 0,05 |
| 95 Wochen 25 °C | 0,16 |
| 156 Wochen 25 °C | 0,43 |

Die Lösung erweist sich als lagerstabil und unempfindlich gegen die Herstellung in Behältern aus Pharmastahl in normalen Produktionsanlagen.

### Beispiel 3: Moxifloxacin-Formulierung 0,04% M/V (40 mg/100 ml)

| | |
|---|---|
| Moxifloxacin-HCl | 0,04 % M/V* |
| Natriumchlorid | 0,9% M/V |
| Wasser f. Injektionszwecke | ad 100 ml |
| Osmolalität: 310 mOsmol/kg | |

| | |
|---|---|
| * Menge bezogen auf das Moxifloxacin in der Betainform | |

### Beispiel 4: Moxifloxacin-Formulierung 0,08% M/V (80 mg/100 ml)

| | |
|---|---|
| Moxifloxacin-HCl | 0,08% M/V * |
| Natriumchlorid | 0,9% M/V |
| Wasser f. Injektionszwecke | ad 100 ml |
| Osmolalität: 312 mOsmol/kg | |

| | |
|---|---|
| * Menge bezogen auf das Moxifloxacin in der Betainform | |

### Beispiel 5: Moxifloxacin-Formulierung 0,2% M/V (200 mg/100 ml),

| | |
|---|---|
| Moxifloxacin-HCl | 0,2% M/V * |
| Natriumchlorid | 0,8% M/V |
| Wasser f. Injektionszwecke | ad 100 ml |
| Osmolalität: 283 mOsmol/kg | |

| | |
|---|---|
| * Menge bezogen auf das Moxifloxacin in der Betainform | |

### Beispiel 6: Moxifloxacin-Formulierung 0,3 % M/V (300 mg/100 ml)

| | |
|---|---|
| Moxifloxacin-HCl | 0,3% M/V * |
| Natriumchlorid | 0,5% M/V |
| Wasser f. Injektionszwecke. | ad 100 ml |
| Osmolalität: 186 mOsmol/kg | |

| | |
|---|---|
| * Menge bezogen auf das Moxifloxacin in der Betainform | |

### Beispiel 7: Moxifloxacin-Formulierung 0,4% M/V (400 mg/100 ml)

| | |
|---|---|
| Moxifloxacin-HCl | 0,4 % M/V* |
| Natriumchlorid | 0,4% M/V |
| Wasser f. Injektionszwecke | ad 100 ml |
| | |
| Osmolalität: 155 mOsmol/kg | |

| | |
|---|---|
| ***** Menge bezogen auf das Moxifloxacin in der Betainform | |

### Beispiel 8 Infusionskonzentrat 2% M/V (400-mg/20ml)

| | |
|---|---|
| Moxifloxacin-Hydrochlorid | 400 mg (berechnet als Betain) |
| Wasser für Injektionszwecke | ad 20 ml |

In einem Mischbehälter aus rostfreiem Stahl pharmazeutischer Qualität wird das Wasser vorgelegt und unter Rühren das Moxifloxacin-Hydrochlorid darin gelöst. Die Lösung wird über einen 0,2 µm Filter filtriert und zu 20 ml in Injektionsflaschen aus Glas abgefüllt, verschlossen und sterilisiert.

Zur Anwendung wird der Inhalt der Injektionsflasche (400 mg Moxifloxacin in 20 ml) mittels einer Spritze entnommen und unter aseptischen Bedingungen zu 230 mL einer handelsüblichen Kochsalzlösung 0,9% gegeben und gemischt. Es entsteht eine isotonische Infusionslösung der Konzentration 400 mg/250 ml, entsprechend 0,16 % M/V. Die Osmolalität beträgt 315 mOsmol/kg.

## Patentansprüche

1. Wäßrige Formulierung, die 0,04 bis 0,4 % M/V Moxifloxacin-Hydrochlorid, berechnet als Moxifloxacin, und 0,4 bis 0,9 % M/V Natriumchlorid enthält.

2. Wäßrige Formulierung nach Anspruch 1, die 0,08 bis 0,32 % M/V Moxifloxacin-Hydrochlorid berechnet als Moxifloxacin, enthält.

3. Wäßrige Formulierung nach einem der vorhergehenden Ansprüche, die 0,1 bis 0,2 % M/V Moxifloxacin-Hydrochlorid, berechnet als Moxifloxacin, enthält.

4. Wäßrige Formulierung nach einem der vorhergehenden Ansprüche, die 0,5 bis 0,9 % M/V Natriumchlorid enthält.

5. Wäßrige Formulierung nach einem der vorhergehenden Ansprüche, die 0,7 bis 0,9 % M/V Natriumchlorid enthält.

6. Verfahren zur Herstellung der wäßrigen Formulierung nach einem der vorhergehenden Ansprüche, wonach zunächst eine Lösung von Moxifloxacin-Hydrochlorid in Wasser hergestellt wird und anschließend Natriumchlorid zugegeben und gelöst wird.

7. Verfahren zur Herstellung der wäßrigen Formulierung nach einem der Ansprüche 1 bis 5, wonach eine Lösung von mehr als 0,4 bis 2,4 % M/V Moxifloxacin-Hydrochlorid, berechnet als Moxifloxacin, in Wasser mit einer wäßrigen Lösung, die Natriumchlorid enthält, vermischt wird.

8. Kombinationspräparat zur Herstellung der wäßrigen Formulierung nach einem der Ansprüche 1 bis 5, das eine wäßrige Lösung von mehr als 0,4 bis 2,4 % M/V Moxifloxacin-Hydrochlorid, berechnet als Moxifloxacin, in Wasser und eine wäßrige Lösung, die Natriumchlorid enthält, getrennt voneinander umfasst.

## Claims

1. Aqueous formulation, comprising from 0.04 to 0.4% w/v. of moxifloxacin hydrochloride, calculated as moxifloxacin, and from 0.4 to 0.9% w/v of sodium chloride.

2. Aqueous formulation according to Claim 1, comprising from 0.08 to 0.32% w/v of moxifloxacin hydrochloride, calculated as moxifloxacin.

3. Aqueous formulation according to either of the preceding claims, comprising from 0.1 to 0.2% w/v of moxifloxacin hydrochloride, calculated as moxifloxacin.

4. Aqueous formulation according to one of the preceding claims, comprising from 0.5 to 0.9% w/v of sodium chloride.

5. Aqueous formulation according to one of the preceding claims, comprising from 0.7 to 0.9% w/v of sodium chloride.

6. Process for preparing the aqueous formulation according to one of the preceding claims, in which a solution of moxifloxacin hydrochloride in water is prepared initially, and sodium chloride is then added and dissolved.

7. Process for preparing the aqueous formulation according to one of Claims 1 to 5, in which a solution of from more than 0.4 to 2.4% w/v of moxifloxacin hydrochloride, calculated as moxifloxacin, in water is mixed with an aqueous solution comprising sodium chloride.

8. Combination preparation for preparing an aqueous formulation according to one of Claims 1 to 5, comprising, separated from one another, an aqueous solution of from more than 0,4 to 2,4% w/v of moxifloxacin hydrochloride, calculated as moxifloxacin, in water with an aqueous solution comprising sodium chloride.

## Revendications

1. Formulation aqueuse qui contient 0,04 à 0,4% M/V de chlorhydrate de moxifloxacine, calculé par rapport à la moxifloxacine, et 0,4 à 0,9% M/V de chlorure de sodium.

2. Formulation aqueuse selon la revendication 1, qui contient 0,08 à 0,32 % M/V de chlorhydrate de moxifloxacine calculé par rapport à la moxifloxacine.

3. Formulation aqueuse selon l'une des revendications précédentes qui contient 0,1 à 0,2% M/V de chlorhydrate de moxifloxacine, calculé. par rapport à la moxifloxacine.

4. Formulation aqueuse selon l'une des revendications précédentes qui contient 0,5 à 0,9% M/V de chlorure de sodium.

5. Formulation aqueuse selon l'une des revendications précédentes qui contient 0,7 à 0,9% M/V de chlorure de sodium.

6. Procédé de préparation de la formulation aqueuse selon l'une des revendications précédentes, selon lequel une solution de chlorhydrate de moxifloxacine est d'abord préparée et le chlorure de sodium est ensuite ajouté et dissous.

7. Procédé de préparation de la formulation aqueuse selon l'une des revendications 1 à 5, selon lequel une solution de plus de 0,4 à 2,4% M/V de chlorhydrate de moxifloxacine, calculé par rapport à la moxifloxacine, est mélangée dans l'eau avec une solution aqueuse qui contient du chlorure de sodium.

8. Préparation combinée pour la préparation de la formulation aqueuse selon l'une des revendications 1 à 5 qui comprend séparément une solution aqueuse de plus de 0,4 à 2,4% M/V de chlorhydrate de moxifloxacine calculé par rapport à la moxifloxavine dans l'eau et une solution aqueuse qui contient du chlorure de sodium.
